# EUROPEAN PATENT APPLICATION

(11) **EP 4 283 297 A1**
(43) Date of publication of application: **29.11.2023**
(21) Application number: 22382512.6
(22) Date of filing: 27.05.2022
(51) Int. Cl.: G01N 33/569, G01N 33/68

(54) **TEST FOR DETECTING CELLULAR IMMUNE RESPONSE TO SARS-COV-2**

(71) Applicant: Institut d'Investigacions Biomèdiques August Pi i Sunyer, 08036 Barcelona (ES); Hospital Clínic de Barcelona, 08036 Barcelona (ES)
(72) Inventor: EGRI CÓRDOBA, Natalia, 08036 BARCELONA (ES); CALDERÓN, Hugo, 08036 BARCELONA (ES); GONZÁLEZ NAVARRO, Europa Azucena, 08036 BARCELONA (ES); HERNÁNDEZ RODRÍGUEZ, José, 08036 BARCELONA (ES); JUAN OTERO, Manel, 08036 BARCELONA (ES)
(74) Representative: ZBM Patents - Zea, Barlocci & Markvardsen

(57) **Abstract**

The present invention provides a quick and reliable method for determining the cellular immune response of a subject to a pathogen by determining the number of antigen-reactive CD4+ T cells by flow cytometry. Also provided are methods for determining the need of a subject for a prophylactic treatment against a pathogen and for diagnosing if a subject has suffered from a pathogen infection based in the cellular immune response of said subject.

## Description

### Technical Field

The present invention refers to the field of clinical analysis. In particular, the invention relates to a method for determining the cellular immune response of a subject to a pathogen.

### Background Art

Severe acute respiratory syndrome coronavirus 2 (SARS-CoV-2), the virus that causes COVID-19 (CoronaVirus Disease 2019), has infected millions of people around the world with an overall mortality rate higher than 2% in patients microbiologically confirmed with COVID-19 before vaccination campaign started. COVID-19 can be manifested with a wide spectrum of disease severity ranging from asymptomatic/mild forms to life-threatening pneumonia with multiple organ failure and death. As soon as vaccines against this virus were available, unprecedented vaccination programs have achieved active immunization of great part of the population in many countries around the world. However, due to the re-emergence of new and more contagious coronavirus variants, the still existence of non-immunized persons, the potential waning of immunity in healthy population and the abnormal immune response in immunocompromised patients, COVID-19 pandemic is expected to remain present for a long time. Pandemic global impact on society is being and will be profound, with special influence in healthcare systems, gender equality and economy.

The relevance of the innate immune response against SARS-CoV-2 is unquestionable. In addition, both humoral and cellular arms of the adaptive immunity are essential towards recovery and protection against coronavirus reinfection. The humoral response opsonizes the free viral particles and promotes both virus elimination and neutralization of the interaction of the virus with their cellular receptors, such as angiotensin converting enzyme 2 (ACE2) and Neuropilin-1. Neutralizing antibodies prevent the virus entry into new cells and avoid the spread of free viral particles. However, antibodies by themselves are unable to clear the virus once it is replicating inside the cells, and the clearance of the established infection exclusively relies on T lymphocytes. T cells are also important to support B lymphocytes functions and antibody affinity maturation. In addition, apart from developing their own function, CD4+ T cells also coordinate humoral responses and assist the cytotoxic activity of CD8+ T cells. In addition, CD8+ T lymphocytes have full capacity to recognize and kill virus-infected cells, hence being the effector component that eliminates intracellular SARS-CoV-2 in COVID-19 patients.

Overall, it is well known that the training of the adaptive immunity by SARS-CoV-2 infection and vaccines are a key component for the resolution of this COVID-19 pandemic. In this regard, several studies have demonstrated that convalescent and vaccinated population develop both humoral and cellular immunity, which significantly protect them from severe disease and death (Krammer F, Correlates of protection from SARS-CoV-2 infection. Lancet. 2021;397(10283):1421-3). Unfortunately, some immunized individuals are known to lose the adaptive protection against the virus due to declining immune response related to advanced age, debilitating chronic diseases, pharmacological immunosuppression or other primary or secondary causes of immunodeficiency (Cucchiari D, et al., Cellular and humoral response after MRNA-1273 SARS-CoV-2 vaccine in kidney transplant recipients. Am J Transplant. 2021;21(8):2727-39). Although less understood, a small proportion of healthy population, especially those with less severe disease forms, never achieve seroconversion while they keep a preserved cellular immunity that protects them against coronavirus(Sekine T, et al., Robust T Cell Immunity in Convalescent Individuals with Asymptomatic or Mild COVID-19. Cell [Internet]. 2020;183(1):158-168.e14; Grifoni A, et al., Targets of T Cell Responses to SARS-CoV-2 Coronavirus in Humans with COVID-19 Disease and Unexposed Individuals. Cell [Internet]. 2020;181(7):1489-1501.e15).

Until now, the evaluation of the immune protection against SARS-CoV-2 has been mainly focused on the detection of antibodies, generally disregarding the cellular response or moving it to second place. However, it is becoming clear that evaluation of the adaptive immunological status against SARS-CoV-2, especially in vulnerable population, may be highly relevant to be able to implement measures to protect those patients with a negative cellular immunization. Moreover, because T-cell responses against SARS-CoV-2 have demonstrated to play a fundamental role in protecting individuals from severe forms of infection, the knowledge of the T-cell immune status may contribute to adjust therapeutic approaches and, importantly, to rationalize active vaccination programs in order to avoid unnecessary expenses and possible side effects of over-immunization.

Unfortunately, in contrast to the simple and automated detection of antibodies, state of the art methods for analyzing the cellular immunity are technically challenging. Assays for detecting T-cell specific responses are long, complex, and labor-intensive. In this sense, gold-standard ELISpots take long time and are technically demanding, since they require from a previous isolation of peripheral blood mononuclear cells (PBMCs).

In view of the above, there is a need in the art for simpler and faster methods for routinely evaluating the adaptive immunological status against SARS-CoV-2.

### Summary of Invention

The present inventors have developed a novel, fast and scalable method to determine the cellular response of a subject to a pathogen, in particular, to a viral pathogen such as SARS-CoV-2. The method is based in detecting reactive T cells from whole blood by flow cytometry and offers reliable results in a working day (less than 8 hours). As shown in the examples below, the results of the method of the invention are comparable with standardized methods, such as ELISpot, albeit obtained significantly faster. The simplicity and speed of the test renders it amenable to routine use in the clinical immunology laboratory, as well as to point-of-care applications.

A first aspect of the invention refers to an *in vitro* method for determining the cellular immune response of a subject to a pathogen, said method comprising:
(i) contacting a sample obtained from a subject with a pathogen antigen (antigen), the sample containing peripheral blood mononuclear cells (PBMCs);
(ii) adding an inhibitor of cytokine secretion;
(iii) optionally lysing red blood cells and removing debris;
(iv) permeabilizing the (non-lysed) cells;
(v) adding at least one fluorescent-labelled CD4+ T cell-defining antibody, at least one fluorescent-labelled cytokine-specific antibody, and at least one fluorescent-labelled antibody which is specific for a T cell activation marker;
(vi) determining the number of antigen-reactive CD4+ T cells by flow cytometry as those CD4+ T cells showing intracellular binding of the cytokine-specific and the T cell activation marker-specific antibody;
wherein a positive cellular immune response to the pathogen is confirmed when the number of determined antigen-reactive T cells is higher than a reference value.

The method enables rapid evaluation of immune protection against potentially aggressive pathogens. Subjects having cellular immune protection against a particular pathogen are considered to entail lower risk of suffering from aggressive disease when infected by the pathogen.

Thus, a second aspect of the invention relates to a method for determining if a subject is protected against severe disease caused by a pathogen, the method comprising determining the cellular immune response of the subject to the pathogen by the *in vitro* method as defined in the first aspect, wherein, if a positive cellular immune response to the pathogen is confirmed, then the subject is considered to be protected.

The routine measurement of the cellular immune response to a particular pathogen afforded by the invention is useful in the clinical practice for deciding on a prophylactic treatment for susceptible subjects, or even for rationalizing active immunization. Rapid knowledge of the immunological status towards a particular pathogen is of great value, in particular, for subjects that do not achieve seroconversion after active immunization against that pathogen, or those losing the adaptive protection against the pathogen due to declining immune response related to time, advanced age, debilitating chronic diseases, pharmacological immunosuppression or other primary or secondary causes of immunodeficiency. In such cases, and many others, subjects that are found to have a positive cellular immune response to the particular pathogen may not require further vaccination. On the contrary, subjects presenting a negative cellular immune response may require prophylactic measures, including vaccination reinforcement and/or other strategies such as isolation, monitorization, etc.

A third aspect of the invention therefore provides a method for determining the need of a subject for a prophylactic treatment against a pathogen, the method comprising determining the cellular immune response of the subject to the pathogen by the *in vitro* method as defined in the first aspect, wherein, if a positive cellular immune response to the pathogen is confirmed, then the subject is rendered not to need the prophylactic treatment.

Finally, because a cellular response seems to be detected in almost all patients who recover from T-cell responses and even in vaccinated patients that have not developed seroconversion, the method of the invention may be used as a diagnostic tool for pathogen infection.

A fourth aspect of the invention thus refers to a method for diagnosing if a subject has suffered or is suffering from a pathogen infection, the method comprising determining the cellular immune response of the subject to the pathogen by the *in vitro* method as defined in the first aspect, wherein, if a positive cellular immune response to the pathogen is determined, then the subject is diagnosed as having suffered or suffering from a pathogen infection.

### Brief Description of Drawings

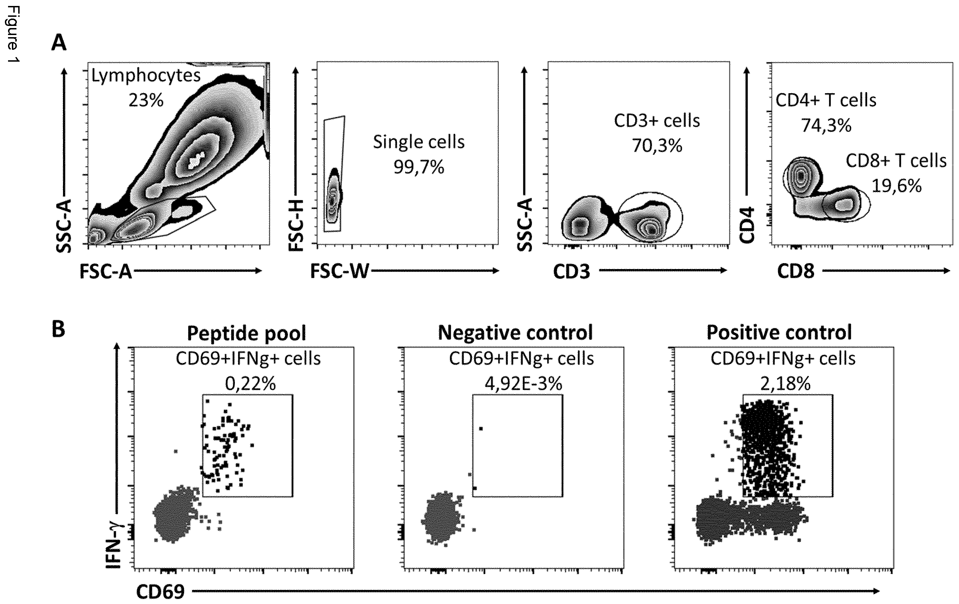

Figure 1. Representative flow cytometry analysis for the CoviTest. The flow cytometry gating strategy was performed sequentially; lymphocyte complexity (SSC vs FSC), single cell, CD3+ and finally either CD4+ or CD8+ (A). From there, 40000 CD4+ or CD8+ T cells were further evaluated for CD69 and IFN-g expression in S and N peptide pool, negative control (vehicle) and positive control (SEB) samples to determine the T cell reactivity for each donor (B).

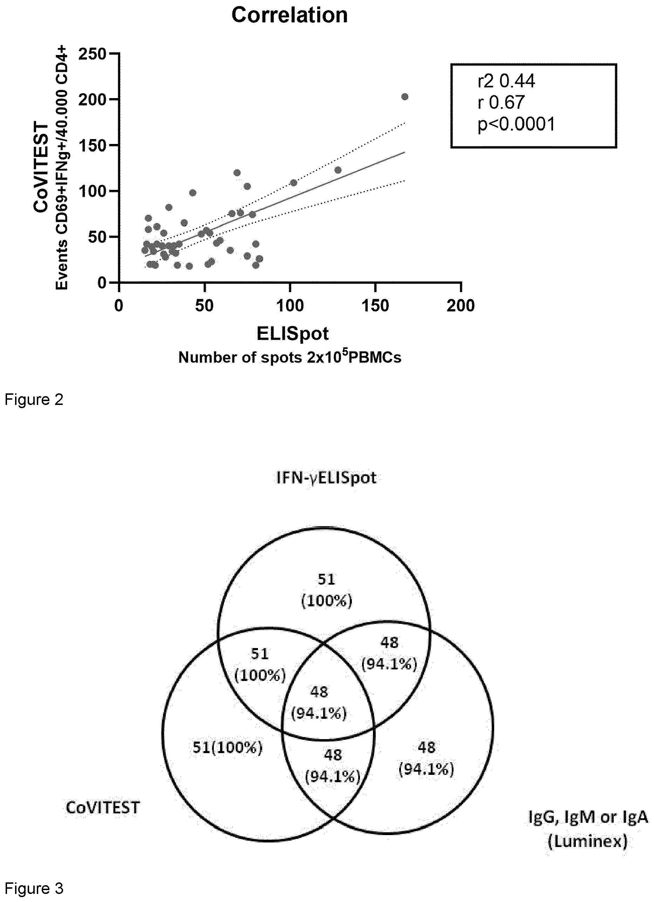

Figure 2. Correlation of SARS-CoV-2-specific T cell response from fresh whole blood CoVITEST with classical T-cell ELISpot-y Convalescent COVID-19 Donors at two weeks,3 and 6 months after the first RT-PCR positive test. Linear regression analysis by comparing the number of T cells CD4+ IFN-γ+ CD69+ from whole blood with specific T cells PBMCs quantified by IFN-γELISpot (n=51).
Figure 3. Distribution of cellular immune response to SARS-CoV-2 by IFN-γ ELISpot, CoVITEST and antibodies against the Receptor Binding Domain (RBD) of the spike glycoprotein of SARS-CoV-2 (IgG, IgA or IgM) by Luminex in 51 COVID-19 patients.

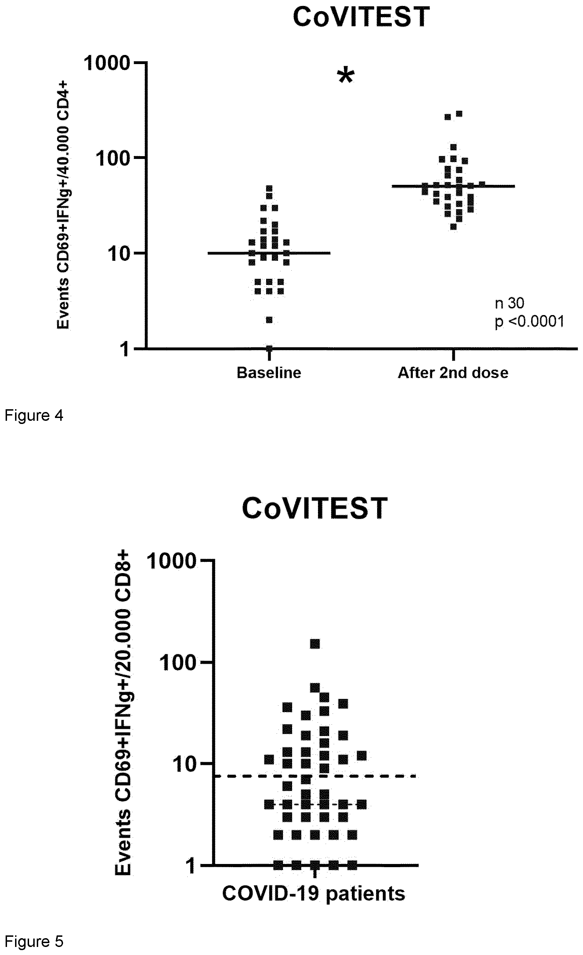

Figure 4. Whole blood (CoVITEST) quantifies SARS-CoV-2-specific T cell response at baseline and 2 weeks after second dose of vaccine in healthy unexposed donors SARS-CoV-2 naïve. SARS-CoV-2 specific T cells (CD4+ IFN-γ+ CD69+) after stimulation with spike and nucleocapsid SARS-CoV-2 peptide pools at baseline and after 2 weeks of second dose of vaccine. Each dot represents an individual subject. Statistical comparison in baseline and post-vaccination were performed with the Wilcoxon test.
Figure 5. Whole blood (CoVITEST) quantifies SARS-CoV-2-specific CD8+ in COVID-19 patientes. SARS-CoV-2 specific T-cells (CD8+ IFN-γ+ CD69+) after stimulation with spike and nucleocapsid SARS-CoV-2 peptide pools two weeks after the first RT-PCR positive test (n=56).

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The definitions given herein are included for the purpose of understanding and expected to be applied throughout description, claims and drawings. It must be noted that, as used herein, the singular forms "a" "an" and "the" include plural referents unless the context clearly dictates otherwise. Thus, for example, reference to "an antigen" includes a single antigen as well as two or more antigens.

The invention described herein provides a sensitive assay to detect antigen specific T cell responses under 8 h, which allows for determining the cellular immune response of a subject to that particular antigen. At its simplest, the method involves contacting a sample containing PBMCs with the antigen specific stimulus and analysing the sample for expression of one or more intracellular cytokines and/or early activation antigens in a particular T cell subset (generally CD4+ T cells). The antigen may be an antigen from a pathogenic organism, for example, a viral antigen, such that the method enables determination of the cellular immune response to that pathogen. The analysis may be done by flow cytometry. In order to detect the desired T cell subset expressing the intracellular cytokines and/or early activation antigens of interest, the method comprises a labelling step where fluorescent-labelled antibodies are contacted with the antigen-stimulated sample.

Thus, the method of the first aspect of the invention requires detecting antigen-reactive T cells, in particular antigen-reactive CD4+ T cells. The procedure for detecting antigen-reactive CD4+ T cells *in vitro* may comprise:
(i) contacting a sample obtained from a subject with a an antigen, the sample containing peripheral blood mononuclear cells (PBMCs);
(ii) adding an inhibitor of cytokine secretion;
(iii) optionally lysing red blood cells and removing debris;
(iv) permeabilizing (non-lysed) the cells;
(v) adding at least one fluorescent-labelled CD4+ T cell-defining antibody, at least one fluorescent-labelled cytokine-specific antibody, and at least one fluorescent-labelled antibody which is specific for a T cell activation marker;
(vi) determining the number of antigen-reactive CD4+ T cells by flow cytometry as those CD4+ T cells showing intracellular binding of the cytokine-specific and the T cell activation marker-specific antibody.

In addition to antigen-reactive CD4+ T cells, the method may simultaneously determine other antigen-reactive T cell subtypes, such as, reactive CD8+ T cells. As outlined above, CD8+ T lymphocytes have full capacity to recognize and kill virus-infected cells. The ability to simultaneously detect and differentiate CD4+ and CD8+ T cell responses is an additional advantage of the present method. For example, knowing whether SARS-CoV-2-reactive CD8+ T cells are present in the sample obtained from patients is useful to provide them with better care. This may be particularly relevant for immunocompromised patients.

In one embodiment of the first aspect of the invention the method further comprises determining antigen-reactive CD8+ T cells by adding in step (v) a fluorescent-labelled CD8+ T cell-defining antibody and determining in step (vi) the number of antigen-reactive CD8+ T cells by flow cytometry as those CD8+ T cells showing intracellular binding of the cytokine-specific and the T cell activation marker-specific antibody. In most embodiments, the method benefits from adding in step (v) a fluorescent-labelled CD3+ T cell-defining antibody. This facilitates the determination of CD4+ and, optionally, of CD8+ T cells.

A CD4+, CD8+, or CD3+ T cell-defining antibody is understood, in the sense of the present invention, as an antibody which specifically binds, respectively, to CD4+ T, CD8+, or CD3+ T cells. Similarly, a cytokine-specific antibody is an antibody that specifically binds to a particular cytokine, for example, IFN-y, and a T cell activation marker-specific antibody is an antibody that specifically binds to a particular T cell activation marker, for example to CD65. Antibodies are well-known binding molecules in the art and are widely used due to their high specificity. However, as will be apparent to the skilled person, the labelling of reactive T-cells may be achieved by using alternative binding molecules, such as antibody fragments, aptamers or any other molecule which may specifically bind to a target of interest and is susceptible of carrying a fluorescent label. Thus, the present invention contemplates using, as alternative to antibodies, any other binding molecule that specifically binds to the targets of interest, in particular, CD4+, CD8+, or CD3+ T cells, CD65 early activation marker, or IFN-y.

In order to determine the cellular response of the subject to the assayed antigen, the number of antigen-reactive CD4+ T cells determined in the sample is compared with a reference value, such that, when the antigen-reactive CD4+ T cells determined in the sample is higher that the reference value, this is indicative of the subject having a cellular immune response to the antigen.

The term "reference value" in the context of the present invention is to be understood as a predefined number of antigen-reactive T cells determined by the method of the invention in a sample of a reference subject or in a group of reference subjects. Preferably, the reference sample is a sample from a naïve subject or a group of naïve subjects. More preferably, the reference value is the average number of antigen-reactive T cells determined by the method of the invention in a group of naïve subjects. A "naïve subject" is understood as someone who has no evidence of having had contact with the pathogen, i.e. has not had the disease caused by said pathogen and was not vaccinated against it. This value may be used to discriminate subjects having a cellular immune response to the antigen from those who don't.

The inventors have also determined an appropriate threshold to discriminate subjects having a cellular immune response to the antigen from those who don't. Thus, in some embodiments, a positive cellular immune response to the pathogen is confirmed when the following criteria are fulfilled:
(a) the number of antigen-reactive CD4+ T cells is equal or above *X* per 40.000 CD4+ T cells, where
   *X* = *R* + *2***SD₁*, wherein
   *R* is the average number of antigen-reactive T cells determined by the method of the invention in a group of naive subjects, and
   *SD₁* is the standard deviation of *R*; *and*
(b) the median fluorescent intensity (MFI) for antigen-reactive CD4+ T cells is equal or above *Y*, where
   *Y* = *F* + *2***SD₂*, wherein
   *F* is the average MFI for antigen-reactive CD4+ T cells in a group of naïve subjects, and *SD₁* is the standard deviation of F.

In particular embodiments, *X* = *R* + *3***SD₁*, more particularly *X = R* + *4*SD₁.* In other particular embodiments, *Y* = *F* + *3*SD₂,* more particularly *Y = F* + *4*SD₂. R* is preferably the average number of antigen-reactive T cells per 40.000 CD4+ T cells determined by the method of the invention in a group of naive subjects

In other particular embodiments, a positive cellular immune response is confirmed when the following criteria are fulfilled in addition to the criteria defined above:
(c) The number of antigen-reactive CD4+ T cells in the test sample is at least 2-fold greater than the number of antigen-reactive CD4+ T cells in an negative control sample per 40.000 CD4+ T cells, wherein the negative control sample is a non-antigen-stimulated sample; and
(d) A positive control sample has more than 100 antigen-reactive CD4+ T cells per 40.000 CD4+ T cells, wherein the positive control sample is a sample which has been stimulated with an antigen known to elicit a strong cellular immune response, for example, staphylococcal enterotoxin B superantigen.

In particular embodiments the number of antigen-reactive CD4+ T cells in the test sample is at least 3-fold greater, more particularly 4-fold greater, than the number of antigen-reactive CD4+ T cells in an negative control sample per 40.000 CD4+ T cells. In particular embodiments, the positive control sample has more than 200 antigen-reactive CD4+ T cells per 40.000 CD4+ T cells. Both negative and positive control samples have the same volume as the test sample and have been obtained from the same subject.

In a very particular embodiment, a positive cellular immune response to the pathogen is confirmed when the following criteria are fulfilled:
(a) 15 or more antigen-reactive CD4+ T cells were detected from 40.000 CD4+ T cells in the test tube.
(b) The MFI for IFN-γ and CD69 on CD4+IFN-γ +CD69+ events was 5000 or above.
(c) The number of antigen-reactive CD4+ T cells in the test tube was at least 2-fold change greater than the unstimulated control tube.
(d) The positive control had more than 100 events.

The antigen may be any antigen or a group of antigens. In particular, the antigen or group of antigens is from a pathogenic organism. Non-limiting examples of pathogens which may be of interest for the present invention are SARS-CoV-2, cytomegalovirus, Epstein-Barr virus, human herpesvirus 6, Mycobacterium spp., BK virus, plasmodium, and Aspergillus fumigatus. In one embodiment, the antigen is a bacterial antigen. In another embodiment, the antigen is a fungal antigen. In another embodiment, the antigen is a viral antigen. In particular embodiments, the antigen is from SARS-CoV-2. Antigens eliciting an immune response from common pathogens are known in the art and are even commercially available. The skilled person may choose the appropriate antigen or group of antigens when wanting to determine the cellular immune response to a pathogen by the method of the first aspect of the invention. In a particular embodiment, the antigen is derived from SARS-CoV-2 nucleocapsid (N) protein or spike (S) protein. In a more particular embodiment, the antigen is a group of antigens derived from SARS-CoV-2 nucleocapsid (N) protein and/or spike (S) protein.

As outlined above, the method of the invention enables to determine the cellular immune response to a particular antigen stimulus. For example, when using SARS-CoV-2 antigens as stimuli in the method of the invention, the cellular immune response to SARS-CoV-2 is determined. The antigen-specific response can be more easily detected when the antigen stimulation is provided in conjunction with costimulation of surface antigens involved with accessory cell surface molecules, such as CD28, CD40L, CD49d, VLA4. Costimuli can be either antibodies or ligands binding to these antigens.

Thus, in one embodiment of the first aspect of the invention, the method further comprises the step of adding to the sample a costimulatory agent of T cell activation concomitant to the antigen. In a particular embodiment the costimulatory agent is directed to CD28 alone or is directed to CD28 and CD49d. In a more particular embodiment, the costimulatory agent is a CD28 antibody. In an even more particular embodiment, the costimulatory agents are a CD28 antibody and a CD49d antibody.

The method of the invention is based on intracellular cytokine detection, which is greatly enhanced by adding an agent which inhibits the secretion of such intracellular cytokines, preferably during the stimulation (activation) phase. Several agents that inhibit the secretion of intracellular cytokines are known in the art and can be used in the present invention. However, in a particular embodiment, the inhibitor is Brefeldin A. Brefeldin A is commercially available.

In another embodiment, the method of the first aspect of the invention further comprises a step of adding to the sample a chelating agent, preferably after adding the inhibitor of cytokine secretion. Chelating agents are employed to release reactive T cells, which may have aggregated, into the analysis buffer. In particular embodiments the chelating agent is a cationic chelating agent, for example EGTA or EDTA. In a more particular embodiment, the chelating agent is EDTA.

In a particular embodiment of the first aspect of the invention the sample containing peripheral blood mononuclear cells is whole blood. Being able to work with whole blood is a relevant advantage of the method of the invention. This is important because isolating PBMCs from whole blood, which is usually the first step for analysing T cell responses, is a time consuming procedure. By working with whole blood directly, the method of the invention may determine T-cell response to a particular antigen is shorter times.

Using whole blood as the sample for the method of the invention usually requires lysing red blood cells. Method for lysing red blood cells are known to the skilled person. In one example, lysis of red blood cells is achieved by adding an erythrocyte lysing solution. The erythrocyte lysing solution may contain, for example, formaldehyde and diethylene glycol, usually around 1% formaldehyde and <5% diethylene glycol. Several lysing solutions are commercially available. In a particular embodiment of the first aspect of the invention, the sample is whole blood. In a particular embodiment, when the sample is whole blood, the method comprises lysing red blood cells by adding an erythrocyte lysing solution, for example, a solution comprising around 1% formaldehyde and <5% diethylene glycol, such as FACS Lysing Solution. In a more particular embodiment, the method further comprises separating non-lysed cells, for example, by centrifuging and decanting.

The method further comprises permeabilizing the PBMCs prior to staining, so as to allow for the cytokine-specific antibodies to reach the target of interest without destroying the cell. Method to permeabilize PBMCs are known to the skilled person. In one embodiment, permeabilization is achieved by using chemicals, for example, by treating the PBMCs with detergents. Several commercially-available permeabilizing solutions may be used in the method of the invention, for example FACS Permeabilizing Solution 2. Thus, in one embodiment of the first aspect of the invention, permeabilization is performed by adding a chemical permeabilization agent, for example, FACS Permeabilizing Solution 2.

Analysis of antigen-reactive T cells is based on the percentage of T cells which express particular markers and/or cytokines, which requires labelling of the cells that express said particular markers and/or cytokines (step v of the method). In one embodiment of the method of the first aspect, antigen reactive T-cells are those expressing CD69 early activation marker and cytokines, in particular, interferon gamma (IFN-γ). Thus, in one embodiment of the first aspect of the invention, labelling in step (v) is done by adding one or more specific antibodies selected from the group consisting of an antibody specific for IFN-y, an antibody specific for CD69, and combinations thereof. In a particular embodiment, the method comprises adding labelled antibody that specifically binds to CD69 and a labelled antibody that specifically binds to IFN-y. Consequently, the method of the invention may comprise determining the number of CD4+ (and optionally CD8-) T cells showing intracellular binding to both CD69-specific and IFN-γ-specific antibodies. When the sample is whole blood, labelling of the cells is done with fixation-compatible antibodies.

Determination of the number of antigen-reactive CD4+ and, optionally, CD8+ T cells, is preferably performed by flow cytometry. Appropriate fluorescent labels for use with the specific antibodies are known in the art. In some embodiments, the fluorescent label is selected from the group consisting of fluorescein isothiocyanate (FITC), phycoerythrin (PE), Peridinin chlorophyll protein (PerCP), and Allophycocyanin (APC). Preferably, each antibody is labelled with a different fluorophore. In particular embodiments, the labelling antibodies are IFN-γ-FITC, CD69-PE, CD3-BV510, CD4-PerCP, and, optionally, CD8-AF700.

The speed of the method of the invention is a very relevant advantage, in particular when compared to alternative methods to determine cellular immunity, such as the gold-standard ELISpots, which are very time consuming. The time required to detect antigen-reactive CD4+ T cells by the method of the invention is less than 24, in particular less than 12 hours, more particularly, less than 8 hours and, even more particularly, less than 6 hours. This means that results for determining the immunological status of a subject with respect to a particular antigen, for example, to a pathogen, by the method of the invention may be obtained in a working day. Thus, in one embodiment of the first aspect of the invention, the time required to determine the cellular immune response of a subject to a pathogen is less than 24 hours, in particular less than 12 hours, more particularly, less than 8 hours and, even more particularly, less than 6 hours. The time required by the method is greatly influenced by the activation step, i.e. by the time for stimulating with the specific antigen. In one embodiment of the present invention, the activation step takes from 3 to 10 hours, or from 4 to 8 hours, for example, around 5 or 6 hours. Regarding temperature conditions, the activation is generally done at physiological temperature (around 37°C) and the remaining steps are performed at room temperature (18-27 °C). The conditions required by each step of the method may vary depending on several circumstances but, as an example, typical conditions are indicated in an exemplary general procedure provided below. The conditions can be extrapolated or adapted when using equivalent reagents to those indicated below.

Exemplary procedure for determining the cellular response to an antigen from whole blood according to the invention:
1. Aliquot whole blood into test tubes;
2. Add antigen and costimulatory agent of T cell activation (e.g. Fastimmune CD28/CD49d cocktail), incubate 1-3 h (e.g. around 2 h) at around 37 °C, around 5% CO2 and around 95% humidity;
3. Add inhibitor of cytokine secretion (e.g. Brefeldin A) and incubate 3 - 6 h (e.g. around 4 h) at around 37°C;
4. Add cation chelator (e.g. EDTA), incubate 5 - 30 min (e.g. 15 min) at room temperature (RT);
5. Add red blood cells lysing solution (e.g. FACS Lysing Solution), incubate 5 - 20 min (e.g. around 10 min) at RT, centrifuge, decant supernatant;
6. Resuspend non-lysed cells in permeabilizing solution (e.g. FACS Permeabilizing Solution 2), incubate 5-20 min (e.g. around 10 min), optionally dilute, centrifuge, decant supernantant;
7. Add to permeabilized cells fluorescent-labelled antibodies (e.g. CD4+ T cell-defining antibody, optionally CD8+ T cell-defining antibody, CD69-specific antibody, and IFN-γ-specific antibody), incubate 15min to 1h (e.g. 30 min) at RT in the dark, centrifuge, decant supernatant;
8. Resuspend labelled cells and analyse by flow cytometry (collect e.g. a minimum of 40,000 CD4+ T cell events).

Washing steps may be performed in between the relevant steps when appropriate. Washing may be done with appropriate buffers (e.g. PBS) or with FACS solutions. All the stimulation, labelling and flow cytometry acquisition procedure can be performed in vented-cap cytometry tubes or, when testing multiple donors at once, a sterile 96 deep-well plate. In the latter case, a flow cytometer fitted with autosampler may be used. Controls are generally included in the analysis to guarantee a correct functioning of the method. Usually, negative control is a non-antigen-stimulated sample from the subject being tested. Positive control may be a sample from the subject being tested which is stimulated with an antigen known to elicit a strong cellular immune response, for example, staphylococcal enterotoxin B superantigen. Control samples have the same volume as test samples and are usually run parallel together with the test samples.

It is to be understood that, while keeping the essential features, the above general procedure may be subject to slight modifications, in particular regarding the reagents and their concentration, temperature and time conditions, as would be apparent to the skilled person.

Because T-cell responses have demonstrated to play a fundamental role in protecting individuals from severe forms of pathogen-associated disease, measurement of the cellular immune response to a particular pathogen according to the method of the invention is useful in the clinical practice, in particular, to adjust therapeutic approaches. For example, according to the test results, patients may benefit from decision-making protocols for re-vaccination against that particular pathogen. In addition, the test of the invention may be used as a diagnostic tool for pathogen infection.

The second, third and fourth aspects of the invention refer to the clinical applications of the method of the invention. All embodiments described above for the method of the first aspect also apply to the second, third and fourth aspects. Moreover, in particular embodiments of any of the first to fourth aspects of the invention, the subject is seronegative for the particular antigen (has not developed a humoral immune response, or wherein the humoral immune response has faded). In another particular embodiment of any of the first to fourth aspects, the subject is an immunocompromised patient. In another particular embodiment of any of the first to fourth aspects, the subject is a healthy subject. In another particular embodiment, the antigen is from SARS-CoV-2. Thus, the first to fourth aspects of the invention may be applied to determining the cellular response to SARS-CoV-2, determining if a subject is protected against severe Covid-19, determining the need of a subject for a prophylactic treatment against a SARS-CoV-2 (e.g. vaccination or re-vaccination), and diagnosing if a subject has suffered or is suffering from a SARS-CoV-2 infection, respectively.

Throughout the description and claims the word "comprise" and variations of the word, are not intended to exclude other technical features, additives, components, or steps. Furthermore, the word "comprise" encompasses the case of "consisting of" and "consisting essentially of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

Herein described is CoVITEST (Covid19 anti-Viral Immunity based in T cells for Evaluation in a Simple Test) as an illustrative example of a method according to the invention to detect cellular immune response from whole blood. It is shown that CoVITEST may be a point-of-care test, based on the detection of T-cell early activation markers and cytokines which offers reliable results in a working day (less than 8 hours), and is also comparable with other standardized methods exploring cellular response to SARS-CoV-2.

### 1. Methods

### 1.1. Human subjects

All participants gave written informed consent, and the inclusions were performed in agreement with Declaration of Helsinski. Approval was obtained from Ethical's Committee of the Hospital Clinic of Barcelona (HCB/2020/0967). The two main tested groups of subjects were:

### (a) Healthy Unexposed Donors

Samples from 30 healthy adult donors were obtained in the Hospital Clinic of Barcelona between October 2020 and April 2021. Subjects were identified to be SARS-CoV-2 naive through self-reporting as well as being double negative for serology and previous cellular response against the virus. The identified subjects were monitored for immunization after vaccination with either Pfizer^{®} (BNT162b2^{™}) or Moderna^{®} (mRNA-1273^{™}) mRNA vaccines, or ChAdOx1n Cov-19^{™} (Oxford-Astrazeneca^{®}).

Blood samples were collected at 2 time-points: pre-vaccine baseline to exclude virus exposure (time-point 1) and two weeks post-second dose vaccination (time-point 2). All participants were otherwise healthy and did not report any history of chronic health conditions. The median age of this control group was 45 (from 22 to 66 years) years.

### (b) Convalescent COVID-19 Donors

Fifty-one convalescent individuals with previous asymptomatic (n=2) or pauci-symptomatic/mild (n=49) COVID-19 were enrolled in the study. Study inclusion criteria were adult subjects (≥18 years) with a positive real-time reverse transcriptase PCR (RT-PCR) test for SARS-CoV-2 on nasopharyngeal swabs at diagnosis. Blood samples were collected two weeks after the first RT-PCR positive test. Median age of the participants was 32.5 (range 23-62) years. All of them were otherwise healthy at the time of the study and did not report any history of chronic health conditions.

### 1.2. Sample collection

Peripheral blood was collected from a forearm vain with butterfly needle coupled to heparinized and serum tube. To compare CoVITEST with gold standard IFN-γELISpot assay, PBMCs were isolated from all collected blood samples by Ficoll-Paque density gradient centrifugation. In each human subject, we analyze samples following workflow described at Figure 1.

### 1.3 CoVITEST (Assay for SARS-CoV-2-specific T-cell evaluation from fresh whole blood).

250 µl freshly isolated whole blood was stimulated with SARS-CoV-2 Spike and Nucleocapside peptide pools (Miltenyi Biotec^{®}) at 50 µg/ml working concentration directly into a sterile tube with a vended-cap. Additionally, negative (PBS with 10% DMSO) and positive (staphylococcal enterotoxin B superantigen at 10 µg/ml working concentration) control tubes were included. Tubes were then spiked with 2.5 µl of Fastimmune^{™} CD28/CD49d cocktail (BD Bioscience^{®}), briefly shake at low speed on a vortex before incubating at 37°C, 5% CO₂ and 95% humidity. After 2 h of incubation, Brefeldin A was added at 1 µg/ml working concentration with a multi-dispenser pipette into all three conditions, briefly shaking at low speed on a vortex before returning the samples to the incubator. 4 hours later, samples were complemented with EDTA (Invitrogen) at 2 mM final concentration, vigorous vortex spin and 15 min incubation at room temperature (RT). Red-blood cells of the samples were lysed, fixing and permeabilizing them with 2.5 mL of 1X BD FACS Lysing Solution (BD Bioscience^{®}), gently mixed and incubate for 10 min at RT. Then centrifuged at 500g for 5 min and decanting later the supernatant. Non-lysed cells were resuspended in 1.25 mL of 1X BD FACS Permeabilizing Solution 2 (BD Bioscience^{®}) and incubated for 10 min at RT. Samples were further diluted with 3.5 mL of wash buffer and centrifuge at 500 g for 5 min before decanting the supernatant.

Cell labelling was performed with fixation-compatible antibodies IFN-γ-FITC (Pharmingen^{®}), CD69-PE, CD8-AF700, CD3-BV510 (BDBioscience^{®}) and CD4-PerCP (Biolegend^{®}) incubated with the permeabilized cells for 30 min at RT in the dark. Following the staining, cells were washed in FACS buffer, centrifuged at 500 g for 5 min; supernatants were decanted before resuspending the cells in 1 ml of FACS buffer. Cells were stored in the dark at 4°C until acquisition on the flow cytometer; samples were analyzed on an NxT Attune Flow Cytometer (ThermoFisher^{®}), evaluating as reactive cells, those CD4+CD3+ T cells, double positive for IFN-γ+ and CD69+.

A positive cellular immune response was confirmed when the results from the test met the following 4 criteria:
- 15 or more events were detected from 40.000 CD4+ T cells in the test tube.
- The MFI for IFN-g+ and CD69+ on CD4 events was 5000 or above.
- The number of events in the test tube was at least 2-fold change greater than the unstimulated control tube.
- The positive control had more than 100 events.

Threshold to reactive test was calculated using the average of IFNg+CD69+events (5.2) plus 2 standard deviations (2*4.7) from pre-vaccine SARS-CoV-2-naive donors. Similarly, the 5000 MFI threshold was established by the MFI average of IFNg+CD69+ events (2846), plus 2 standard deviations (2*1197).

All the stimulation, staining and flow cytometry acquisition procedure can be performed in a sterile 96 deep-well plate instead of vented-cap cytometry tubes when testing multiple donors at once (use a flow cytometer fitted with autosampler). When assessing multiple donors, peptide pools and controls can be prepared in master mix with Fastlmmune cocktail and aliquoted with multi-dispenser pipette.

### 1.4 IFN-γELISpot

Stimulation was conducted with 2 × 10⁵ PBMCs in X-VIVO^{™} 15 medium (Lonza^{®}) supplemented with 10% heat inactivated AB serum and PepTivator^{®} SARS-CoV-2 Prot_S and N peptide pools1 (1 µg/ml, Miltenyi Biotec^{®}). The diluent was PBS+DMSO with final concentration of DMSO 1%. In the negative control of the ELISpot, the X-VIVO 15 medium was employed with DMSO 20% to a final concentration of 1%. Negative control wells lacked peptides, and positive control wells included mAb CD3-2 of Kit. Cells were incubated overnight (16-20h) at 37°C 5% CO2 in precoated anti-IFN-y MSIP white plates (mAb 1-D1K, Mabtech^{®}).

Plates were then washed 5-times with PBS (Sigma-Aldrich) and incubated for 2 h at RT with horseradish peroxidase (HRP)-conjugated anti-IFN-y detection antibody (1 µg/ml; clone mAb-7B6-1; Mabtech^{®}). After five further washes with PBS, tetramethylbenzidine (TMB) substrate was added and spots were counted using an automated ELISpot Reader System (Autoimmun Diagnostika GmbH^{®}).

To quantify positive peptide-specific responses, spots of the unstimulated wells were subtracted from the peptide-stimulated wells and the results expressed as SFU (Spot Forming Units) /2×10⁵ PBMCs.

We determined SARS-CoV-2-specific spots by spot increment, defined as stimulated spot numbers ≥6 SFU/2 × 10⁵ PBMCs. This cutoff was defined calculating the mean +/- 2 standard deviations in a group of healthy donors obtained prior to the start of the pandemic of SARS-CoV-2. Spot counting was done automatically and re-evaluated manually in all cases.

### 1.5 Quantification of antibodies to SARS-CoV-2 by Luminex^{®}

To establish seroprevalence, we used a serological assay based on the Luminex^{®} technique that has the benefit of a higher dynamic range than other assays, favoring the quantification of immunoglobulin levels. We measured antibodies against the Receptor-Binding Domain (RBD) of the spike glycoprotein of SARS-CoV-2 by Luminex^{®}(14). Median fluorescent intensities (MFI) were exported using the xPONENT^{™} software. Assay cutoff was calculated as the mean plus 2 standard deviations of log10-transformed MFIs of a donor pool of 30 negative samples obtained before the COVID-19 pandemic. The data used for the calculations were the ratio of the MFI of the particular individual with the raw MFI obtained from the donor pool, and a value ≥1 was considered to be positive.

### 1.6 Statistic analysis

All statistical analyses were performed using GraphPad Prism, version 8 (GraphPad^{®} Software). Significant differences in each group were analyzed by the Wilcoxon matched-pairs signed-rank- test. Were applicable, the statistical tests used, and the definition of median are indicated in the figure legends. Statistical significance was set at a p value of less than 0.05. In all instances, n refers to the number of patients analyzed.

### 2. Results

### 2.1. Assessment of the SARS-CoV-2-specific T cell response directly from fresh whole blood (CoVITEST) results comparable to those obtained with gold standard assays (IFN-γELISpot).

We determined the presence of SARS-CoV-2 specific T-cell in 51 mild COVID-19 with IFN-γELISpot and with CoVITEST, by analyzing samples collected 2 weeks, 3 months and 6 months after the diagnostic infection to SARS-CoV-2 to define the reliability of CoVITEST for quantification of the SARS-CoV-specific T-cell responses. We then compared our results from CoVITEST with those from IFN- γ ELISpot. We found a good correlation (r = 0.67 p<0.0001) of CoVITEST in whole blood that remained well correlated with IFN- γ ELISpot assays (Figure 2).

These results indicate that the CoVITEST, which utilizes freshly collected whole blood, is a feasible method for reliable quantification of SARS-CoV-2-specific T-cell responses, producing results that are comparable to those obtained with well-established assays used to analyze T-cell responses.

### 2.2. Distribution the cellular and humoral immune response in COVID-19 patients.

We analyzed the distribution de cellular and humoral response in 51 COVID-19 patients, 94.1% had cellular and humoral immune response to SARS-CoV-2 detected by all three IFN-ELISpot, CoVITEST and antibodies detection by Luminex (IgG, IgA or IgM), 100% had cellular immune response to SARS-CoV-2 by IFN-γELISpot and CoVITEST (sensibility 100%) and finally 94.1% had cellular and humoral immune response to SARS-CoV-2 by CoVITEST and Luminex (Figure 3).

Also, we analyze SARS-CoV-2-specific CD8+ in COVID-19 patients (n=51) with CoVITEST, 41% develop CD8+ responses (Supplementary Material, Figure 5).

### 2.3.Rapid quantification of SARS-CoV-2-specific T cells by CoVITEST

We characterized the initial kinetics of specific T cells induced by 2 doses of the mRNA vaccine BNT162b2, mRNA-1273 and ChAdOx1 nCoV-19 (University Oxford/Aztrazeneca) in 30 healthy individuals at baseline and 2 weeks after second dose of vaccine SARS-CoV-2-specific T cells were seen in 100% of healthy individuals at 2 weeks after second doses (Figure 4).

### 3. Discussion

Developing worldwide T-cell protection against the virus probably is a key factor to resolve COVID-19 pandemic. In this regard, it is known that individuals without any underlying chronic disease in whom an acquired and protective cellular immunity against SARS-CoV-2 has been detected, do not always develop antibody seroconversion. This baseline cellular response has been associated with complete recovery from asymptomatic or mild forms of the disease (12). Furthermore, patients unable to produce antibodies (because of inherited or treatment-mediated B-cell deficiencies) have been also reported to recover from COVID-19(15). In part, this might be explained in accordance to what happens in patients with hematological malignancies, in whom CD8+ T cells seem to compensate the lack of humoral immunity and contribute to improve outcomes of COVID-19 patients. These facts strongly support the role of T cells in protection against SARS-CoV-2 infection and are also relevant for other infections.

In the same sense, Cucchiari *et al* studied cellular and humoral responses in 117 SARS-CoV-2-naïve kidney transplant recipients after receiving mRNA-1273 vaccine (Moderna)(3). After two vaccine doses, 35 (29.9%) patients developed either IgG or IgM and 64 (54.7%) patients developed cellular responses to the coronavirus(3). Additionally, 50% of the 82 patients with baseline doble negativity anti-SARS-CoV-2 IgG and IgM developed T-cell immunity against the virus(3). Therefore, the status of the immune response against SARS-CoV-2, either by a protection triggered by passive immunization or after vaccinations, especially in immunosuppressed patients, must be evaluated by the study of both reactive T cells and antibody levels.

Although T-cell responses are detected in almost all patients who recover from COVID-19, not all COVID-19 and SARS-CoV-2 vaccinated patients have detectable CD8+ specific T-cells (12). In this regard, two studies reported that 100% and 89% of convalescent COVID-19 patients had CD4 T-cell reactivity, while only 70% and 69% of them developed CD8+ responses, respectively(13). Because CoVITEST offers the ability to differentiate CD4+ from CD8+ T-cell responses, our CoVITEST data are aligned with those previously evidenced, since CD8+ T-cell responses are substantially less frequently observed than CD4+ lymphocytes (Figure 2 and Figure 4).

Based on our results with patients infected with SARS-CoV-2, it is plausible that CoVITEST could become a reliable and rapid platform used to detect reactive T cells against other viruses, including cytomegalovirus, Epstein-Barr virus, and human herpesvirus 6, Mycobacterium spp., and different fungi, such as *Aspergillus fumigatus.* This would be especially relevant in patients after allogeneic hematopoietic stem cell and solid organ transplantation, since it is well known that post-transplant therapies include cyclophosphamide and other immunosuppressive agents, and they are associated with an increased rate of opportunistic infections. Because the immunity of transplant patients with neoplastic conditions is generally assessed by long-lasting and expensive techniques, our CoVITEST assay could be similarly used to rapidly evaluate the immune protection against potentially aggressive pathogens in these fragile patients. Extrapolation of the CoVITEST to detect reactive T cells against other viruses only requires substituting SARS-CoV-2 antigens by relevant antigens from the other viruses.

Because T-cell responses against SARS-CoV-2 have demonstrated to play a fundamental role in protecting individuals from severe forms of infection, the knowledge of the T-cell immune status may contribute to adjust therapeutic approaches, particularly in patients with an abnormally reduced function of the immune system. With this aim, we created CoVITEST, a rapid, simple and accurate in-house method for routine measurement of SARS-CoV-2 reactive T cells from whole blood of selected individuals. As a result, in both SARS-CoV-2 convalescent individuals and healthy-vaccinated donors, CoVITEST demonstrated to provides accurate results in a working day, comparable to those obtained from the gold standard IFN-γELISpot assays, which analyze cellular immunity in a longer amount of time (Figure 2 and 3).

Apart from the detection of specific SARS-CoV-2 T cells, CoVITEST also offers the ability to differentiate CD4+ from CD8+ T-cell responses. This is an additional advantage over ELISpot, since knowing whether SARS-CoV-2-reactive CD8+ T cells are present may be relevant to provide a better care to immunocompromised patients. In addition, CoVITEST could be also of utility for diagnostic purposes in patients with previous seronegative (or never done) tests and suspected COVID-19-related conditions (e.g. long-COVID-19 symptoms, post-viral thrombotic complications or interstitial lung diseases, etc.) in whom previous positive tests for SARS-CoV-2 diagnosis were never obtained or performed.

In conclusion, CoVITEST is a novel in-house method that provides a rapid measurement of specific SARS-CoV-2 T cells in whole blood in a simple and affordable manner. It can reliably detect spike-specific T cell (differentiating CD4+ from CD8+ T-cell) responses induced after SARS-CoV-2 infection or vaccination, and also saves time compared to the standardized IFN-γELISpot assay. Patients with a suspected or confirmed immunosuppression may benefit from decision-making protocols for re-vaccination against SARS-CoV-2 derived from CoVITEST results. In addition, CoVITEST may be used as a diagnostic tool for SARS-CoV-2 infection in particular clinical situations.

### Citation List

Cucchiari D, Egri N, Bodro M, Herrera S, Del Risco-Zevallos J, Casals-Urquiza J, et al. Cellular and humoral response after MRNA-1273 SARS-CoV-2 vaccine in kidney transplant recipients. Am J Transplant. 2021;21(8):2727-39.
Krammer F. Correlates of protection from SARS-CoV-2 infection. Lancet. 2021;397(10283):1421-3.
Sekine T, Perez-Potti A, Rivera-Ballesteros O, Strålin K, Gorin JB, Olsson A, et al. Robust T Cell Immunity in Convalescent Individuals with Asymptomatic or Mild COVID-19. Cell [Internet]. 2020;183(1):158-168.e14. Available from:
   https://doi.org/10.1016/j.cell.2020.08.017
Grifoni A, Weiskopf D, Ramirez SI, Mateus J, Dan JM, Moderbacher CR, et al. Targets of T Cell Responses to SARS-CoV-2 Coronavirus in Humans with COVID-19 Disease and Unexposed Individuals. Cell [Internet]. 2020;181(7):1489-1501.e15. Available from: http://dx.doi.org/10.1016/j.cell.2020.05.015
Soresina A, Moratto D, Chiarini M, Paolillo C, Baresi G, Focà E, et al. Two X-linked agammaglobulinemia patients develop pneumonia as COVID-19 manifestation but recover. Pediatr Allergy Immunol. 2020;31(5):565-9.

## Claims

**1.** An *in vitro* method for determining the cellular immune response of a subject to a pathogen, said method comprising:
(i) contacting a sample obtained from a subject with a pathogen antigen (antigen), the sample containing peripheral blood mononuclear cells (PBMCs);
(ii) adding an inhibitor of cytokine secretion;
(iii) optionally lysing red blood cells and removing debris;
(iv) permeabilizing the cells;
(v) adding at least one fluorescent-labelled CD4+ T cell-defining antibody, at least one fluorescent-labelled cytokine-specific antibody, and at least one fluorescent-labelled antibody which is specific for a T cell activation marker;
(vi) determining the number of antigen-reactive CD4+ T cells by flow cytometry as those CD4+ T cells showing intracellular binding of the cytokine-specific and the T cell activation marker-specific antibody;
wherein a positive cellular immune response to the pathogen is confirmed when the number of determined antigen-reactive T cells is higher than a reference value.

**2.** The *in vitro* method according to claim 1, wherein a positive cellular immune response to the pathogen is confirmed when the following criteria are fulfilled:
(a) the number of antigen-reactive CD4+ T cells is equal or above *X* per 40.000 CD4+ T cells, where
*X* = *R* + *2***SD₁*, wherein
*R* is the average number of antigen-reactive T cells determined by the method of the invention in a group of naive subjects, and
*SD₁* is the standard deviation of *R*; *and*
(b) the median fluorescent intensity (MFI) for antigen-reactive CD4+ T cells is equal or above *Y*, where
*Y* = *F* + *2***SD₂*, wherein
*F* is the average MFI for antigen-reactive CD4+ T cells in a group of naïve subjects, and *SD₁* is the standard deviation of F.

**3.** The *in vitro* method according to claim 2, wherein a positive cellular immune response is confirmed when the following criteria are further fulfilled:
(c) The number of antigen-reactive CD4+ T cells in the test sample is at least 2-fold greater than the number of antigen-reactive CD4+ T cells in an negative control sample per 40.000 CD4+ T cells, wherein the negative control sample is a non-antigen-stimulated sample; and
(d) A positive control sample has more than 100 antigen-reactive CD4+ T cells per 40.000 CD4+ T cells, wherein the positive control sample is a sample which has been stimulated with an antigen known to elicit a strong cellular immune response, for example, staphylococcal enterotoxin B superantigen.

**4.** The *in vitro* method according to any one of claims 1-3, wherein the antigen is a viral antigen, in particular a SARS-CoV-2 antigen.

**5.** The *in vitro* method according to any one of claims 1-4, further comprising determining antigen-reactive CD8+ T cells by adding in step (v) a fluorescent-labelled CD8+ T cell-defining antibody and determining in step (vi) the number of antigen-reactive CD8+ T cells by flow cytometry as those CD8+ T cells showing intracellular binding of the cytokine-specific antibody and the T cell activation marker-specific antibody.

**6.** The *in vitro* method according to any one of claims 1-5, wherein the cytokine-specific antibody is an antibody specific for IFN-y and the T cell activation marker-specific antibody is an antibody specific for CD69.

**7.** The *in vitro* method according to any one of claims 1-6, further comprising adding in step (v) a fluorescent-labelled CD3+ T cell-defining antibody.

**8.** The *in vitro* method according to any one of claims 1-7, further comprising the step of adding to the sample a costimulatory agent of T cell activation, in particular, a CD28 antibody, concomitant to the antigen.

**9.** The *in vitro* method according to any one of claims 1-8, further comprising the step of adding to the sample a chelating agent, preferably after adding the inhibitor of cytokine secretion and before permeabilizing.

**10.** The *in vitro* method according to any one of claims 1-9, wherein the sample containing peripheral blood mononuclear cells is whole blood.

**12.** The *in vitro* method according to any one of claims 1-11, wherein the fluorescent labels are selected from the group consisting of fluorescein isothiocyanate (FITC), phycoerythrin (PE), peridinin chlorophyll protein (PerCP), and allophycocyanin (APC).

**13.** A method for determining the need of a subject for a prophylactic treatment against a pathogen, in particular determining the need of vaccination, the method comprising determining the cellular immune response of the subject to the pathogen by the *in vitro* method as defined in any one of claims 1-12, wherein, if a positive cellular immune response to the pathogen is determined, then the subject is considered not to need the prophylactic treatment.

**14.** A method for diagnosing if a subject has suffered or is suffering from a pathogen infection, the method comprising determining the cellular immune response of the subject to the pathogen by the *in vitro* method as defined in any one of claims 1-12, wherein, if a positive cellular immune response to the pathogen is determined, then the subject is diagnosed as having suffered or suffering from a pathogen infection.

**15.** The method according to any one of claims 13 or 14, wherein the pathogen is SARS-CoV-2.
